(19) Europäisches Patentamt
European Patent Office
Office européen des brevets

(11) Veröffentlichungsnummer : **0 281 832 B1**

# (12) EUROPÄISCHE PATENTSCHRIFT

(45) Veröffentlichungstag der Patentschrift : **02.05.91 Patentblatt 91/18**

(51) Int. Cl.$^5$ : **C07F 9/547,** A61K 31/675

(21) Anmeldenummer : **88102612.4**

(22) Anmeldetag : **23.02.88**

## (54) Verfahren zur Herstellung von Ifosfamid mit verbesserten Eigenschaften.

Die Akte enthält technische Angaben, die nach dem Eingang der Anmeldung eingereicht wurden und die nicht in dieser Patentschrift enthalten sind.

(30) Priorität : **06.03.87 DE 3707154**

(43) Veröffentlichungstag der Anmeldung : **14.09.88 Patentblatt 88/37**

(45) Bekanntmachung des Hinweises auf die Patenterteilung : **02.05.91 Patentblatt 91/18**

(84) Benannte Vertragsstaaten : **AT BE CH DE ES FR GB GR IT LI LU NL SE**

(56) Entgegenhaltungen : **FR-M- 6 695**

(73) Patentinhaber : **ASTA Pharma Aktiengesellschaft**
**Weismüllerstrasse 45**
**W-6000 Frankfurt am Main 1 (DE)**

(72) Erfinder : **Engel, Jürgen, Dr.**
**Erlenweg 3**
**W-8755 Alzenau (DE)**
Erfinder : **Müller, Siegfried**
**Platanenring 10**
**W-6380 Bad Homburg (DE)**
Erfinder : **Laubner, Werner**
**Haartstrasse 2**
**W-4802 Halle (DE)**

EP 0 281 832 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Der chemische Name für den Wirkstoff Ifosfamid ist 3-(2-Chlorethyl)-2-(chlorethylamino)-tetrahydro-2H-1,3,2-oxazaphosphorin-2-oxid

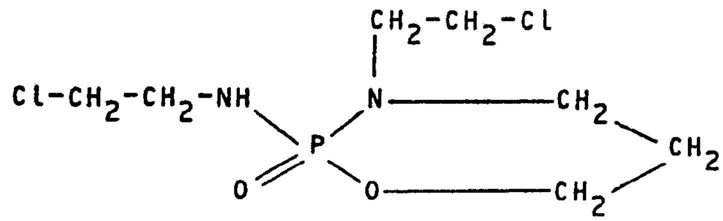

Ifosfamid gehört wie Cyclophosphamid zur chemischen Gruppe der Oxazaphosphorine und wird therapeutisch zur Behandlung von Tumor-Erkrankungen eingesetzt.

Ifosfamid ist ein weißes, kristallines Pulver mit einem Schmelzpunkt von 48°C bis 51°C und stark hygroskopischen Eigenschaften. Bereits unterhalb des Schmelzpunktes beginnt Ifosfamid zu sintern ; es muß deshalb bei möglichst niedrigen Temperaturen (Raumtemperatur und darunter) gelagert werden. Außerdem ist ein Kontakt mit Luftfeuchtigkeit möglichst zu vermeiden.

Das zur Zeit erhältliche Ifosfamid neigt zum Verkleben und Verklumpen sowie zur elektrostatischen Aufladung. Dadurch ist die genaue Dosierung beziehungsweise Abfüllung in Injektionsflaschen sehr schwierig beziehungsweise überhaupt nicht möglich.

Außerdem ist die Qualität der Produktionschargen schwankend.

Aufgabe der Erfindung ist daher die Herstellung von Ifosfamid, das die geschilderten Nachteile nicht mehr aufweist, und insbesondere bessere Abfülleigenschaften besitzt.

Diese Aufgabe wird erfindungsgemäß durch kontrolliertes Umkristallisieren von Ifosfamid in einem bestimmten Lösungsmittel-Gemisch gelöst.

Dieses Lösungsmittel besteht entweder aus einer Mischung von Diethylether und einem $C_1$-$C_3$-Alkanol, vorzugsweise Diethylether/Methanol oder einer Mischung von Diisopropylether und einem $C_1$-$C_3$-Alkanol, vorzugsweise Diisopropylether/Methanol, Diisopropylether/Isopropanol oder Diisopropylether/n-Propanol.

Das Verhältnis des $C_1$-$C_3$-Alkanols zu dem Ether kann dabei zwischen 1 : 1 bis 1 : 200, vorzugsweise 1 : 5 bis 1 : 200 liegen (Volumen pro Volumen). Die in Frage kommenden $C_1$-$C_3$-Alkohole sind : Methanol, Ethanol, Propanol und Isopropanol.

Bei dem bisher bekannten Ifosfamid sind die Einzelkristalle meist unvollständig ausgebildet mit abgerundeten Kanten und zeigen eine stark ausgeprägte Agglomerat- beziehungsweise Aggregatbildung. Mindestens 70% der Kristalle des bisher bekannten Ifosfamids (zum Beispiel 70-80%) weisen eine Partikelgröße zwischen 100 bis 180 µm auf, wobei das Verhältnis Länge zu Breite dieser Kristalle in dem Bereich von 3,0 : 1 bis 3,8 : 1 liegt. Weiterhin hat dieses vorbekannte Ifosfamid einen hohen Staubanteil (das heißt Partikel von 10-50 µm).

Außerdem hat das bisher bekannte Ifosfamid sehr schlechte Abfülleigenschaften (das heißt die Dosierungsgenauigkeit ist mangelhaft). Die Abweichung der Einzelwerte vom Sollwert bei Dosierungsprüfungen einzelner Produktionschargen liegt zum Beispiel zwischen 0,9 und 6,1%, woraus sich eine durchschnittliche Standardabweichung von 2,29% ergibt. (Bestimmung der Standardabweichung nach Münzel, Büchi, Schultz, Galenisches Praktikum, Wissenschaftliche Verlagsgesellschaft mbH, Stuttgart, 1959).

Demgegenüber weist das erfindungsgemäß hergestellte Ifosfamid eine durchschnittliche relative Standardabweichung von maximal 1,4% auf (Abweichung der Einzelwerte vom Sollwert bei Abfüllprüfungen zwischen 0,90% und 2%). Ganz wesentlich ist hierbei außerdem die geringe Streuung der Einzelwerte.

Die Dosierungsprüfungen (Abfüllprüfungen) erfolgen beispielsweise volumetrisch mittels einer Schnecken-Abfüllmaschine bestehend aus einem Sammelbehälter mit eingepaßtem Rührwerk (sorgt für eine gleichmäßige Verteilung der Substanz), wobei dem Sammelbehälter ein größerer Vorratsbehälter vorgeschaltet ist, aus dem mit Hilfe eines Zuteilmessers im Takt des Abfüllens eine entsprechende Menge Substanz – grob vordosiert – zugeteilt wird. Hierdurch wird gewährleistet, daß das Gemisch der auf der Schnecke lastenden Füllgutsäule stets gleichmäßig bleibt und keine Schwankungen der Säulenhöhe eintreten.

Die Dosierung erfolgt mittels einer Schnecke. Diese Schnecke befindet sich in der unteren öffnung (Austrittsdüse) des hier spitz zulaufenden Sammelbehälters.

Um ein gleichmäßiges Gefüge innerhalb des Dosierungsraumes zu gewährleisten, erweitert sich die Schnecke nach oben innerhalb des Sammelbehälters, wobei gleichzeitig die Steigung der Schneckengänge steiler wird, so daß die Schnecke stets mehr Substanz erfaßt als sie dosieren kann, wodurch zwangsläufig die

etwa noch eingeschlossene Luft entweicht. Ein Haftenbleiben von Substanz an den Wänden des Dosierraums wird dadurch vermieden, daß der Dosierraum zylindrisch ist, und die Schnecke mit äußerster, mechanisch noch zulässiger Toleranz eingepaßt ist. Die Schnecke macht während eines Abfüllvorganges eine jeweils bestimmte und konstante einstellbare Zahl von Umdrehungen. Diese Umdrehungszahl wird über einen Potentiometer (0,3-3,0 Sekunden), der die Dauer des Dosiervorganges reguliert, eingestellt.

Diese Umdrehungszahl und die zugehörige Größe der Schnecke hängt von der jeweils abzufüllenden Menge (Dosierungsmenge) ab. Die entsprechenden Parameter gehen aus der folgenden Tabelle hervor :

Tabelle

| Dosierungsmenge | Schnecken-Nr. | Bohrung der Austrittsdüse | Abfüllzeit pro Dosierung*) |
|---|---|---|---|
| 200 mg | 10 | 4 mm | ca. 1,6 sec. |
| 500 mg | 23 | 6 mm | ca. 1,1 sec. |
| 1 g | 32 | 8 mm | ca. 1,5 sec. |
| 2 g | 33 | 8 mm | ca. 1,3 sec. |

*) Die Abfüllzeiten schwanken leicht von Charge zu Charge und müssen jeweils genau eingestellt werden.

Um das Nachrieseln zu verhindern, ist die Austrittsdüse des Sammelbehälters verjüngt, und zwar bis auf 0,5 mm. Durch die Oberflächenspannung kann ein Nachrieseln nicht mehr stattfinden.

Die Abfüllung geschieht in rhythmischer Folge. Die theoretisch mögliche Leistung beträgt zum Beispiel 50 Abfüllungen in der Minute.

Eine vorstehend beschriebene Abfüllmaschine ist zum Beispiel die Penicillin-Abfüllmaschine vom Typ DOS Mikro der Firma Höfliger und Karg, Spezialfabrik für automatische Waagen und Verpackungsmaschinen, Waiblingen bei Stuttgart, Heerstraße, Bundesrepublik Deutschland.

Der pH-Wert einer 10%igen (Gewichtsprozent) wäßrigen Lösung des erfindungsgemäßen Ifosfamids liegt bei 5,5 bis 6 (die erlaubten Grenzwerte sind 4,5 bis 6,5) und besitzt daher ohne zusätzliche Maßnahmen die erforderliche Eignung für die therapeutische Anwendung als Injektionslösung.

Das erfindungsgemäß erhaltene Ifosfamid besteht aus nadelförmigen prismatischen Kristallen (meist langgestreckt) mit einer maximalen Partikelgröße bis 450-500 µm, vereinzelt bis 700 µm oder sogar 960 µm.

Die kleinste Partikelgröße liegt beispielsweise bei 10-20 µm.

Mindestens 70% der vorliegenden Kristalle (zum Beispiel 70-80%) weisen eine Partikelgröße zwischen 80-450 µm beziehungsweise 80-550 µm auf, beispielsweise zwischen 80-350 µm oder zwischen 100-350 µm beziehungsweise 80-300 µm. Das Verhältnis von Länge zu Breite der Kristalle beträgt von 2,5 : 1 bis 9,5 : 1 insbesondere von 3,0 : 1 bis 7,5 : 1, wobei dieses Verhältnis bei mindestens 70% der vorliegenden Kristalle (zum Beispiel 70-80%) in einem Bereich von 3 : 1 bis 7,5 : 1 liegt (zum Beispiel von 4,0 : 1 bis 7,5 : 1 oder 3,5: 1 bis 6,5 : 1).

Die Kristalle des erfindungsgemäßen Ifosfamids sind gut ausgebildet und weisen keine oder nur geringfügig abgerundete Kanten auf. Der Staubanteil (das heißt Partikel mit einer Größe zwischen 10-50 µm) ist gering.

Eine Aggregatbildung ist wenig bis kaum vorhanden.

Es wird keine elektrostatische Aufladung der Kristalle zum Beispiel beim Abfüllen beobachtet.

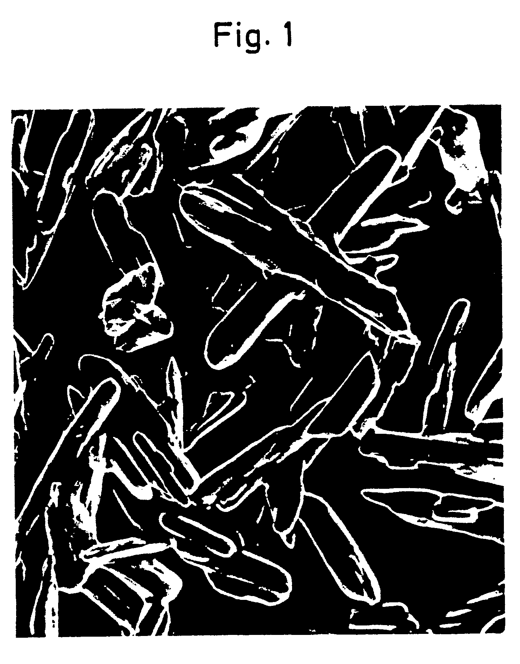

Figur 1 zeigt die Aufnahme des erfindungsgemäßen Ifosfamids unter dem Raster-Elektronenmikroskop

(REM) bei einer Vergrößerung 100 : 1.

Es ist überraschend, daß nur durch die Umkristallisation in dem erfindungsgemäßen Lösungsmittelgemisch ein Ifosfamid mit stark verbesserten Eigenschaften erhalten werden kann, da entsprechende Versuche unter Verwendung zahlreicher anderer üblicher Lösungsmittel und Lösungsmittelgemische nicht zum Ziel geführt haben. Beispielsweise haben Versuche mit folgenden Lösungsmitteln nicht zum Erfolg geführt : Wasser, Alkohole ($C_1$-$C_4$), Tetrahydrofuran, Dioxan, 1,2-Dimethoxyethan, Dichlormethan, Chloroform, 1,2-Dichlorethan, 1,1,1-Trichlorethan, Bromethan, Methylacetat, Methylformiat, Ethylformiat, 1,1,2-Trichlortrifluorethan, Aceton, Ethylmethylketon, Isobutylmethylketon, flüssige niedere Kohlenwasserstoffe Gemische von chlorierten Kohlenwasserstoffen und Pentan, Gemische von Ketonen und Pentan, Alkohol-Pentan-Gemische, Ether-Pentan-Gemische, tert.-Butylmethylether,
2-Chlorpropan, 1-Chlorpropan, 1-Chlorbutan-Methanol-Gemische, 2-Chlorbutan-Methanol-Gemische, 1-Chlor-2-methylpropan-Methanol-Gemische, 2-Chlor-2-methylpropan-Methanol-Gemische, 1-Chlorpentan-Methanol-Gemische, 2-Chlor-2-methylbutan-Methanol-Gemische, Fluorbenzol, Toluol, Propylformiat, n-Butylformiat, n-Butylformiat-n-Butanol-Gemische, Isobutylformiat, Isopentylformiat, Ethylacetat, Isopropylacetat, n-Propylacetat, Isobutylacetat, tert.-Butylacetat, Isopropenylacetat, Methylpropionat, Ethylpropionat, Isopropylpropionat, Gemische von Diethylether-Methanol-isopropanolischer Salzsäure, Gemische von 2-Chlor-2-methylpropan und Triethylamin.

Bei dem für das erfindungsgemäße Verfahren einzusetzenden Ifosfamid handelt es sich um das zur Zeit erhältliche Ifosfamid. Die Herstellung dieses Ifosfamids erfolgt zum Beispiel nach dem Verfahren der deutschen Patentschrift 1 645 921.

Die verwendeten Lösungsmittel sollen möglichst trocken sein. Die Trocknung dieser Mittel kann zum Beispiel erfolgen durch Destillation oder andere übliche Trockenmittel wie Molekularsiebe, Magnesiumsulfat, Natriumsulfat, Calciumchlorid.

Die Lösung des Ifosfamids in dem erfindungsgemäßen Lösungsmittel-Gemisch erfolgt bei einer Temperatur zwischen 25 und 30°C unter Feuchtigkeitsverschluß, zweckmäßig unter Rühren (30 bis 500, vorzugsweise 100 bis 200 Umdrehungen pro Minute) oder durch Schütteln. Die Lösung soll gerade gesättigt beziehungsweise mehrfach übersättigt sein.

Das einzusetzende Ifosfamid kann in kristalliner Form oder auch in teilweise zerschmolzener Form vorliegen.

Auf 100 g Ifosfamid werden zum Beispiel 100 bis 2000, vorzugsweise 100 bis 1000, insbesondere 150 bis 700 oder auch 200 bis 500 ml des erfindungsgemäßen Lösungsmittel-Gemisches verwendet. Dabei liegt das Volumen-Verhältnis des $C_1$-$C_3$-Alkanols zu dem betreffenden Ether zwischen 1 : 1 bis 1 : 200 vorzugsweise zwischen 1 : 5 bis 1 : 200.

Im einzelnen richtet sich das Verhältnis Alkanol zu Ether auch nach der jeweils verwendeten Gesamtmenge des erfindungsgemäßen Lösungsmittel-Gemisches. Beispielsweise kommen hier folgende Verhältnisse von Alkanol zu Ether in Abhängigkeit von der Lösungsmittelmenge pro 100 g Ifosfamid in Frage (der Temperaturbereich, in dem bei solchen Mischungen beispielsweise angeimpft werden kann, ist in Spalte 3 angegeben) :

| ml Lösungsmittel-Gemisch pro 100 g Ifosfamid | Verhältnis $C_1$-$C_3$-Alkanol zu Ether | Impftemperatur |
|---|---|---|
| 100 - 2000 | 1:1 bis 1:200 | -20 bis +25°C |
| 100 - 1000 | 1:2 bis 1:150 | -10 bis +23°C |
| 150 - 700 | 1:4 bis 1:120 | 0 bis +21°C |
| 200 - 500 | 1:5 bis 1:100 | +5 bis +20°C |

Das Volumenverhältnis des $C_1$-$C_3$-Alkanols zu dem jeweils verwendeten Ether richtet sich im einzelnen auch danach welche spezielle Alkanol-Ether-Mischung verwendet wird. In der Tabelle 1 sind entsprechende

Volumenverhältnisse für die einzelnen Alkanol-Ether-Mischungen beispielhaft aufgeführt, sowie die bevorzugten entsprechenden Temperaturbereiche, in denen angeimpft werden kann. Bei den jeweils mit b, c und d gekennzeichneten Bereichen handelt es sich um bevorzugte Bereiche (die Buchstaben a-d gelten stets für die ganze Zeile).

Tabelle 1

| Lösungsmittel | ml Lösungsmittel-Gemisch pro 100 g Ifosfamid | Verhältnis (ml) Alkanol zu Ether | Impftemperatur |
|---|---|---|---|
| Diethylether/Methanol | a) 100 - 2000 | 1: 1 bis 1:150 | -20 bis +25 |
| | b) 150 - 600 | 1: 2 bis 1:100 | 0 bis +23 |
| | c) 200 - 350 | 1: 5 bis 1: 70 | +10 bis +20 |
| | d) 230 - 300 | 1:10 bis 1: 50 | +17 bis +20 |
| Diethylether/Ethanol | a) 100 - 2000 | 1: 1 bis 1.150 | -20 bis +25 |
| | b) 100 - 1000 | 1: 2 bis 1:100 | 0 bis +23 |
| | c) 150 - 600 | 1: 5 bis 1: 70 | +10 bis +21 |
| | d) 200 - 350 | 1:10 bis 1: 50 | +15 bis +20 |

| Lösungsmittel | ml Lösungsmittel-Gemisch pro 100 g Ifosfamid | Verhältnis (ml) Alkanol zu Ether | Impftemperatur |
|---|---|---|---|
| Diethylether/Isopropanol (und auch n-Propanol) | a) 100 - 2000 | 1: 1 bis 1:200 | -20 bis +25 |
| | b) 200 - 1000 | 1: 5 bis 1:150 | 0 bis +20 |
| | c) 250 - 700 | 1:10 bis 1:120 | + 5 bis +15 |
| | d) 300 - 450 | 1:25 bis 1:100 | + 8 bis +12 |
| Diisopropylether/Methanol | a) 100 - 2000 | 1: 1 bis 1:150 | -20 bis +25 |
| | b) 100 - 1000 | 1: 2 bis 1:100 | -10 bis +20 |
| | c) 150 - 700 | 1: 4 bis 1: 50 | 0 bis +18 |
| | d) 200 - 500 | 1: 7 bis 1: 25 | + 5 bis +15 |

| Lösungsmittel | ml Lösungsmittel-Gemisch pro 100 g Ifosfamid | Verhältnis (ml) Alkanol zu Ether | Impftemperatur |
|---|---|---|---|
| Diisopropylether/ Ethanol | a) 100 - 2000 | 1: 1 bis 1:200 | -20 bis +25 |
| | b) 150 - 1000 | 1: 3 bis 1:150 | -10 bis +20 |
| | c) 200 - 700 | 1: 5 bis 1:100 | 0 bis +18 |
| | d) 250 - 500 | 1:10 bis 1: 75 | +5 bis +15 |
| Diisopropylether/ Isopropanol | a) 100 - 2000 | 1: 1 bis 1:200 | -20 bis +25 |
| (und auch n-Propanol) | b) 200 - 1000 | 1: 5 bis 1:150 | 0 bis +20 |
| | c) 250 - 700 | 1:10 bis 1:120 | +5 bis +15 |
| | d) 300 - 450 | 1:25 bis 1:100 | +8 bis +12 |

Die Lösung des Ifosfamids in der Ether-Methanol-Mischung kann zusätzlich mit Aktivkohle und/oder amorpher Kieselsäure behandelt werden. Auf 1 Gewichtsteil Ifosfamid werden 0,005 bis 0,05, vorzugsweise 0,01 bis 0,02 Gewichtsteile verwendet. Die Behandlung mit Aktivkohle sowie mit der amorphen Kieselsäure erfolgt, um eventuell vorhandene harzartige Verfärbungen zu entfernen.

Als Aktivkohle kommt zum Beispiel in Frage : Tierkohlen sowie aktivierte Pflanzen-Holzkohlen, die durch reine Wasserdampfaktivierung hergestellt und gegebenenfalls durch Nachbehandlung mit Mineralsäure und entionisiertem Wasser auf einen sehr niedrigen Aschegehalt gebracht werden, das heißt vegetabilische Aktivkohlen, die sich bei geringem Gesamtaschegehalt durch niedrige Schwermetallgehalte auszeichnen. Solche Kohlepulver erfüllen zum Beispiel die Anforderungen des DAB 8 (West) (DAB = Deutsches Arzneibuch, 8. Ausgabe von 1978) beziehungsweise der Europäischen Pharmakopöe 2. Polycyclische Kohlenwasserstoffe sind innerhalb der vorgeschriebenen Grenzen nicht nachweisbar. Geeignet sind zum Beispiel Medizinalkohle-Pulver nach DAB 8 (West) mit folgender Porenverteilung :
Mikroporen (Durchmesser 0-20 Angström) : 0,6 ml/g
Mesoporen (Durchmesser 20-300 Angström) : 0,15 ml/g
Makroporen (Durchmesser größer als 300 Angström) : 0,5 ml/g.
Weiterhin kommen gewaschene (das heißt mit Mineralsäure und entionisiertem Wasser nachbehandelte) und gepulverte Entfärbungs-Aktivkohlen in Frage, die hinsichtlich Aschegehalt, Schwermetallgehalt und fluoreszierender Stoffe die Anforderungen des DAB 8 erfüllen und nicht so feinporig wie die Medizinalkohle sind. Derartige Entfärbungs-Aktivkohlen besitzen beispielsweise folgende Porenverteilung :
Mikroporen : 0,2-0,4 ml/g, insbesondere 0,4 ml/g
Mesoporen : 0,2-0,5 ml/g, insbesondere 0,2 ml/g
Makroporen : ca. 0,5 ml/g, insbesondere 0,5 ml/g.
Solche Entfärbungs-Aktivkohlen werden beispielsweise von der Degussa Aktiengesellschaft hergestellt und sind unter den geschützen Bezeichnungen EPONIT (zum Beispiel EPONIT 114Spezial, EPONIT 113Spezial, mit Phosphorsäure neutralisierte Kohlen wie EPONIT 113 Np, EPONIT 114 Np), Carbopuron und Degusorb im Handel.

Auf 1 Gewichtsanteil Ifosfamid werden 0,005 bis 0,05, vorzugsweise 0,01 bis 0,02 Gewichtsteile amorphe Kieselsäure verwendet.
Als amorphe Kieselsäure kommt zum Beispiel in Frage : Kieselgur, Kieselgele, wie zum Beispiel synthetisch hergestellte, hochporöse amorphe Kieselsäure in Form von harten Körnern mit einer Körnung von 0,15 bis 10 mm. Besonders günstig ist eine Körnung von 0,15 bis 0,30 mm. Der Wassergehalt kann beispielsweise bis zu 10% betragen. Die spezifische Oberfläche kann bis 650 $m^2/g$ betragen. Im allgemeinen liegt sie etwa bei 400 $m^2/g$. Das Schüttgewicht kann bis 650 g/l betragen. Günstig ist ein Schüttgewicht von 450 bis 500 g/l.

Insbesondere wird Kieselgur verwendet. Die Behandlung mit Aktivkohle und der amorphen Kieselsäure erfolgt bei 20 bis 40, vorzugsweise 25 bis 30°C, während 5 bis 60, vorzugsweise 10 bis 40, insbesondere 15 bis 20 Minuten unter Bewegung der Ifosfamid-Lösung, zum Beispiel durch Rühren oder Schütteln.

Nachdem die Ifosfamid-Lösung von den Adsorbentien abgetrennt wurde, zum Beispiel durch Filtration wird die Lösung auf die Impftemperatur gebracht, vorzugsweise mit einer Innentemperatur, die oberhalb 11°C liegt, insbesondere zwischen 14 und 20°C. Diese Abkühlung erfolgt in bekannter Weise zum Beispiel mittels Methanol/Trockeneis oder Thermostaten in einer Zeit von 10 bis 600, vorzugsweise 20 bis 120 Minuten.

Das Animpfen der so erhaltenen Ifosfamid-Lösung erfolgt mit reinem Ifosfamid, wobei auf 100 Gewichtsteile 0,01 bis 5, vorzugsweise 0,1 bis 0,5 Gewichtsteile Impfkristalle verwendet werden.

Das zum Impfen verwendete Ifosfamid kann zum Beispiel gemäß der deutschen Patentschrift 1 645 921 (Beispiel 4) erhalten werden. Wichtig ist, daß das zum Impfen verwendete Ifosfamid völlig kristallin ist oder zumindest deutlich kristalline Anteile enthält.
Reine Impfkristalle können zum Beispiel auch dadurch erhalten werden, daß man von der Ifosfamid-Lösung in dem erfindungsgemäßen Lösungsmittelgemisch eine Probe in ein Glasgefäß überführt, abkühlt (zum Beispiel auf 0°C) und gelegentlich mit dem Glasstab anreibt, wobei gegebenenfalls etwas reiner Diethylether zugegeben wird. Abscheidung der Kristalle erfolgt dann beispielsweise innerhalb von 30 Minuten bis zu einer Woche.

Nach dem Animpfen wird die Lösung unter ständiger Bewegung (zum Beispiel Rühren mit einer Umdrehungsgeschwindigkeit von 30 bis 150, vorzugsweise 50 bis 80 Umdrehungen/Minute) 1 bis 72, vorzugsweise 3 bis 48, insbesondere 8 bis 24 Stunden bei einer Innentemperatur von – 20 bis + 25, vorzugsweise +5 bis + 20, insbesondere + 11 bis + 20°C oder + 14 bis + 20°C gehalten und anschließend gegebenenfalls langsam und gleichmäßig das heißt kontrolliert, unter ständiger Bewegung beziehungsweise ständigem Rühren auf – 10 bis + 5°C, vorzugsweise – 5 bis +5°C insbesondere – 5 bis 0°C abgekühlt.

Diese Abkühlung erfolgt durch entsprechende gleichmäßige Temperaturerniedrigung des Kühlbades, beispielsweise in einem Zeitraum von 8 bis 72, vorzugsweise 8 bis 30 Stunden. Die Abkühlung erfolgt linear, beispielsweise mit Hilfe eines programmierten Thermostates.

Nach dem Animpfen beginnt das Ifosfamid auszukristallisieren. Die Rührgeschwindigkeit nach dem Animpfen und auch während der anschließenden Abkühlung soll so eingestellt werden, daß sich die gebildeten Kristalle nicht zu stark am Boden absetzen. Das Rühren beziehungsweise die Bewegung soll so sein, daß die Kristalle gerade noch in der Schwebe gehalten werden, sich also nicht absetzen.

Das Abfiltrieren der ausgeschiedenen Ifosfamid-Kristalle erfolgt zweckmäßig unter inerter Atmosphäre und unter Ausschluß von Feuchtigkeit, zum Beispiel unter einem trocknenen Inertgas (zum Beispiel Stickstoff, Edelgase wie Argon oder auch Luft mit einer relativen Feuchte von unter 25%) und unter einem Druck von beispielsweise 0,1 bis 3 bar.

Es empfiehlt sich, durch die abgesaugten Ifosfamid-Kristalle nach Waschen mit trockenem Diethylether oder einem anderen Mittel (wie zum Beispiel Kohlenwasserstoffen mit 5 bis 7 C-Atomen), 0,1 bis 10, vorzugsweise 0,5 bis 2 Stunden ein trockenes Inertgas (wie zuvor angegeben) zu blasen, wobei die Kristalle etwa die gleiche Temperatur haben sollen, auf die sie vorher abgekühlt worden waren. Gegebenenfalls kann eine Temperaturerhöhung bis zu 10°C in Kauf genommen werden. Diese Temperatur kann zum Beispiel dadurch eingestellt werden, daß die Filtereinrichtung auf die zuvor angegebene Temperatur gekühlt wird und/oder daß das Inertgas auf diese Temperatur gekühlt wird.

Die Entfernung des Restlösungsmittels erfolgt in schonender Weise bei einer Temperatur zwischen 0 und 40, vorzugsweise 10 und 25°C unter Vakuum von 0,1 bis 100, vorzugsweise 1 bis 20 mbar, wobei die Kristallmasse in langsamer Bewegung gehalten wird, zum Beispiel durch langsames oder gelegentliches Umrühren (zum Beispiel 6 bis 20 Umdrehungen/Minute). Bei Verwendung eines Rotationsverdampfers kann dies zum Beispiel dadurch erreicht werden, daß man diesen während der ersten 6 Stunden in jeder Stunde 1 bis 2 Minuten lang drehen läßt und anschließend nur noch innerhalb von 2 bis 3 Stunden für 1 bis 2 Minuten die Rotation anstellt.

Die Zeit dieser Trocknung beträgt zum Beispiel 5 bis 100, vorzugsweise 20 bis 50 Stunden.

Beispiel 1

1400 g Ifosfamid (Rohprodukt, hergestellt gemäß Beispiel 4 des deutschen Patents 1 645 921), werden unter Rühren und Feuchtigkeitsausschluß in einer Mischung aus 3280 ml getrocknetem Diethylether und 80 ml Methanol (pro analysi) bei 26°C gelöst (Lösung ist trüb). Nun werden 13 g Aktivkohle und 6,5 g Kieselgur zugesetzt und 60 Minuten bei 27-30°C gerührt. Anschließend werden die Adsorbentien über eine Drucknutsche abfiltriert (Lösung trüb) und die Mutterlauge in ein 6 Liter Planschliffreaktionsgefäß (mit eingepaßtem Ankerrührer, Thermometer, Steigrohr mit Trockenrohr, Kühlmantel und Ablaßspindel am Gefäßboden) überführt, auf eine Innentemperatur von 17,1°C abgekühlt (Kühlbadtemperatur 17°C), die Lösung bei einer Rührerumdrehung von 80 Umdrehungen/Minute mit 5 g kristallinem Ifosfamid* angeimpft, 24 Stunden bei 17°C Innentemperatur gerührt und dann gleichmäßig während 24 Stunden auf 0°C abgekühlt, wobei das Ifosfamid auskristallisiert. Die so erhaltenen Kristalle werden auf ein abgekühltes Druckfilter (0°C) gegeben und mit Stickstoff abgedrückt und mit 2000 ml auf 0°C abgekühltem Diethylether 2mal nachgewaschen ; anschließend wird 1 Stunde Stickstoff durch die Filterschicht geblasen (Temperatur der Drucknutsche 0°C). Die Kristalle werden dann in einen 6 Liter Birnenkolben überführt und am Rotationsverdampfer bei Raumtemperatur während 30 Stunden bei 6 Umdrehungen/Minute (gelegentlich für 1-2 Minuten angestellt) und 10-20 mbar getrocknet.

Ausbeute : 1204 g (86% der Theorie)

* erhalten gemäß Beispiel 4 des deutschen Patents 1 645 921

Das so erhaltene Ifosfamid hat nadelförmig prismatische Kristallform ; maximalen Partikelgröße bis 480 µm, vereinzelt bis 700 µm, wobei 70 bis 80% der vorliegenden Kristalle eine Partikelgröße zwischen 150-350 µm haben. Die Proportionen der Kristalle (Länge : Breite) sind : von 3,0 : 1 bis 7,5 (oder auch 9,3) : 1. Bei 70 bis 80% der vorliegenden Kristalle ist das Verhältnis von Länge zu Breite von 3,5 : 1 bis 8,0 : 1.

Die Kristalle sind gut ausgebildet (nur geringfügig abgerundete Kanten). Der Staubanteil ist gering.

Beispiel 2

60 kg Ifosfamid (Rohprodukt) werden bei 30°C in 160 Liter Diethylether und 10 Liter Methanol gelöst. Nach Zugabe von 600 g Aktivkohle (Eponit) wird verrührt. Anschließend wird der Ansatz filtriert und wie folgt kristallisiert :

– In 1,5 bis 2,0 Stunden unter Rühren auf 17°C abgekühlt, dann mit 200 g Ifosfamid beimpft.

Temperatur von 17°C wird 12 Stunden gehalten, danach Absenkung der Temperatur innerhalb 10 Stunden auf 15°C. Linearer Temperaturabfall auf 0°C innerhalb von 18 Stunden. Temperatur von 0°C wird 6 Stunden gehalten.

– Die Rührgeschwindigkeit während dieser Vorgänge wird nach folgendem Schema verändert:
die ersten 4 Stunden bei 32 Umdrehungen/Minute
dann ungefähr 2 Stunden bei 42 Umdrehungen/Minute
dann ungefähr 14 Stunden bei 55 Umdrehungen/Minute
dann ungefähr 28 Stunden bei 65 Umdrehungen/Minute.

Das Kristallisat wird dann in einen Aufgußfilter überführt und die Mutterlauge mit Stickstoff abgedrückt. Danach wird zweimal mit je 20 Liter Diethylether gespült, das Lösungsmittel entfernt und abschließend 1 Stunde mit Stickstoff trockengedrückt. Die Substanz wird in einen Mischtrockner eingetragen und nach folgender Methode getrocknet:

Ungefähr 3 Stunden Vortrocknung im Wasserstrahlpumpen-Vakuum.
Mischer auf Dauerbetrieb.
Ungefähr 45 Stunden Endtrocknung im Ölpumpen-Vakuum.
Mischer auf Intervallbetrieb (Dauer des Intervalls 5 Sekunden/Stunde).

Ausbeute: 46 kg ungefähr 77% ohne Mutterlaugensubstanz Schmelzpunkt 48,9°C

Abfüllfähigkeit (Standardabweichung) = 0,94% (Gute Abfüllfähigkeit bei Standardabweichungen von 1,0 bis 1,5%).

Beispiel 3

1400 g Ifosfamid (Rohprodukt) werden in einer Mischung aus 3280 ml getrocknetem Diisopropylether und 400 ml trockenem Methanol bei 30°C gelöst. Zu der Lösung werden 10 g Aktivkohle zugesetzt und 60 Minuten bei 30°C gerührt.

Über eine Drucknutsche wird die Aktivkohle abfiltriert, und die Mutterlauge in ein 6-Liter-Planschliff-Reaktionsgefäß mit eingepaßtem Ankerrührer, Thermometer, Steigrohr mit Trockenrohr, Kühlmantel und Ablaßspindel am Gefäßboden überführt.

Die Lösung wird dann bei einer Rührerumdrehung von 80 Umdrehungen/Minute innerhalb von 15 Stunden auf 5°C abgekühlt und bei dieser Temperatur mit 10 g kristallinem Ifosfamid angeimpft (langsam beginnende Kristallisation). Es wird 5 Stunden bei 5°C Innentemperatur gerührt, anschließend gleichmäßig linear während einer Stunde auf 0°C abgekühlt und 36 Stunden bei 0°C gerührt.

Das auskristallisierte Ifosfamid wird auf ein abgekühltes Druckfilter (von 0°C) gegeben und mit Stickstoff abgedrückt. Es wird mit 2000 ml auf 0°C gekühltem trockenen Isopropylether 2mal nachgewaschen. Anschließend wird 1 Stunde lang Stickstoff durch die Filterschicht geblasen (Temperatur der Drucknutsche 0°C).

Die Kristalle werden dann in einem 6-Liter-Birnenkolben am Rotationsverdampfer bei Raumtemperatur während 36 Stunden getrocknet unter einem Druck von 10-20 mbar (Drehung des Rotationsverdampfers 6 Umdrehungen pro Minute, gelegentlich für 1 bis 2 Minuten angestellt).

Die Ausbeute beträgt 795,8 g (rund 57% der Theorie).

Der Schmelzpunkt des so erhaltenen Ifosfamids liegt bei 49-50°C.

Beispiel 4

46 kg Ifosfamid (Rohprodukt) werden in einer Mischung aus 160 l Diisopropylether und 2 l Isopropanol bei 27°C gelöst. Es wird dann innerhalb von 3 Stunden auf +10°C abgekühlt und bei dieser Temperatur mit 0,16 kg kristallinem Ifosfamid angeimpft. Die Mischung wird nun für 16 Stunden bei einer Temperatur zwischen 7 und 10°C gerührt (50 Umdrehungen/Minute). Dann wird abgekühlt innerhalb von 10 Stunden linear und gleichmäßig auf 0°C.

Wie in den vorangegangenen Beispiel beschrieben wird das Ifosfamid abgesaugt, gewaschen und mit Stickstoff begast und im Mischtrockner das Lösungsmittel entfernt.

Schmelzpunkt: 49°C
Ausbeute: 35,5 kg = 72,2%

## Ansprüche

1. Nadelförmig prismatisches kristallines Ifosfamid gekennzeichnet durch folgende Eigenschaften:
a) Die Partikelgröße von mindestens 70% der Kristalle liegt in dem Bereich zwischen 150-550 µm mit einem Verhältnis von Länge zu Breite der Einzelkristalle von 3,5 : 1 bis 8 : 1.
b) Die relative Standardabweichung ist maximal 1,4%.
c) Der pH-Wert einer 10%igen (Gewichtsprozent) wäßrigen Lösung beträgt 5,5 bis 6.

2. Verfahren zur Herstellung von Ifosfamid mit verbesserten Eigenschaften, dadurch gekennzeichnet, daß man eine Lösung von 100 g Ifosfamid in 100 bis 2000 ml eines Lösungsmittelgemisches aus Diethylether und einem $C_1$-$C_3$-Alkanol oder Diisopropylether und einem $C_1$-$C_3$-Alkanol, wobei das Volumenverhältnis des $C_1$-$C_3$-Alkanols zu dem Ether jeweils 1 : 1 bis 1 : 200 ist, bei einer Temperatur zwischen −20 bis +25°C unter Rühren mit reinem Ifosfamid animpft, mehrere Stunden bei der Impftemperatur weiterrührt, gegebenenfalls unter Rühren innerhalb von 8 bis 48 Stunden gleichmäßig auf 0 °C bis −5°C abkühlt (falls bei einer Raumtemperatur oberhalb 0°C angeimpft wurde) und die erhaltenen Kristalle isoliert und trocknet.

3. Verfahren nach Anspruch 2, dadurch gekennzeichnet, daß als Lösungsmittelgemisch ein Diethylether-Methanol-Gemisch verwendet wird.

4. Verfahren nach einem oder mehreren der vorangegangenen Ansprüche, dadurch gekennzeichnet, daß das Animpfen oberhalb von 11°C erfolgt.

5. Verfahren nach einem oder mehreren der vorangegangenen Ansprüche, dadurch gekennzeichnet, daß das Animpfen zwischen 14 und 20°C erfolgt.

6. Verfahren nach einem oder mehreren der vorangegangenen Ansprüche, dadurch gekennzeichnet, daß die Abkühlung von der Impftemperatur auf die Filtriertemperatur durch kontrollierte, gleichmäßige lineare Temperaturerniedrigung des Kühlbades erfolgt.

7. Ifosfamid, erhältlich nach einem oder mehreren der vorangegangenen Verfahrensansprüche.

8. Verwendung von Ifosfamid nach einem 1 oder 7 der Ansprüche zur Herstellung von Arzneimitteln.

## Claims

1. Acicular, prismatic, crystalline ifosfamide, characterized by the following properties :

a) the particle size of at least 70% of the crystals is in the range from 150 to 550 µm with a length-to-width ratio of the individual, crystals of 3.5 : 1 to 8 : 1 ;

b) the relative standard deviation is at most 1.4% ;

c) the pH value of a 10% (by weight) aqueous solution is 5.5 to 6.

2. A process for the preparation of ifosfamide having improved properties, characterized in that a solution of 100 g ifosfamide in 100 to 2,000 ml of a solvent mixture of diethyl ether and a $C_{1-3}$ alkanol or diisopropyl ether and a $C_{1-3}$ alkanol, the ratio by volume of the $C_{1-3}$ alkanol to the ether being 1 : 1 to 1 : 200, is seeded while stirring pith pure ifosfamide at a temperature of −20 to +25°C, stirred for several hours at the seeding temperature, uniformly cooled, optionally while stirring, to 0°C to −5°C over a period of 8 to 48 hours (where seeding was carried out at a room temperature above 0°C) and the crystals obtained are isolated and dried.

3. A process as claimed in claim 2, characterized in that a mixture of diethyl ether and methanol is used as the solvent mixture.

4. A process as claimed in one or more of the preceding claims, characterized in that seeding is carried out above 11°C.

5. A process as claimed in one or more of the preceding claims, characterized in that seeding is carried out between 14 and 20°C.

6. A process as claimed in one or more of the preceding claims, characterized in that cooling from the seeding temperature to the filtration temperature is carried out by controlled, uniform linear temperature reduction of the cooling bath.

7. Ifosfamide obtainable in accordance with one or more of the preceding process claims.

8. The use of the ifosfamide according to claim 1 or 7 for the production of medicaments.

## Revendications

1. Ifosfamide en cristaux prismatiques en aiguilles, caractérisé par les propriétés suivantes :

a) la grosseur de particules d'au moins 70% des cristaux se situe dans la gamme comprise entre 150 et 550 µm, avec un rapport de la longueur à la largeur des cristaux individuels de 3,5 : 1 à 8 : 1.

b) l'ecart-type relatif est au maximum de 1,4%.

c) le pH d'une solution aqueuse à 10% (en poids) se situe entre 5,5 et 6.

2. Procédé de préparation d'ifosfamide ayant des propriétés améliorées, caractérisé en ce qu'on ensemence avec de l'ifosfamide pur, à une température comprise entre −20 et +25 °C et sous agitation, une solution de 100 g d'ifosfamide dans 100 à 2000 ml d'un mélange de solvants composé de diéthyléther et d'un alcanol en $C_1$ à $C_3$ ou de diisopropylether et d'un alcanol en $C_1$ à $C_3$, le rapport volumique de l'alcanol en $C_1$ à $C_3$ à l'éther étant compris chaque fois entre 1 : 1 et 1 : 200, on maintient l'agitation pendant plusieurs heures à la

température d'ensemencement, on refroidit éventuellement de façon uniforme à une température de 0 °C à –5 °C sous agitation en l'espace de 8 à 48 h (dans le cas où l'ensemencement a été effectué à une température ambiante supérieure à 0°C), et on isole et on sèche les cristaux obtenus.

3. Procédé selon la revendication 2, caractérisé en ce qu'un mélange de diéthyléther et de méthanol est utilisé comme mélange de solvant.

4. Procédé selon la revendication 2 ou 3, caractérisé en ce que l'ensemencement est effectué au-dessus de 11°C.

5. Procédé selon l'une quelconque des revendications 2 à 5, caractérisé en ce que l'ensemencemnt est effectué entre 14 et 20°C.

6. Procédé selon l'une quelconque des revendications 2 à 5, caractérisé en ce que le refroidissement de la température d'ensemencement à la température de filtration est effectué par abaissement linéaire uniforme contrôlé de la température du bain réfrigérant.

7. Ifosfamide, pouvant être obtenu selon l'une quelconque des revendications de procédé 2 à 6.

8. Utilisation d'ifosfamide selon la revendication 1 ou 7 pour la préparation de médicaments.

Fig. 1